# EUROPEAN PATENT SPECIFICATION

(11) **EP 0 592 454 B1**
(45) Date of publication and mention of the grant of the patent: **28.08.1996**
(21) Application number: 92908854.0
(22) Date of filing: 23.04.1992
(51) Int. Cl.: A61K 39/02

(54) **PRODUCTION OF VACCINES**
IMPFSTOFFERZEUGUNG
PRODUCTION DE VACCINS

(30) Priority: 23.04.1991 GB 9108682
(43) Date of publication of application: 20.04.1994
(73) Proprietor: MALLINCKRODT VETERINARY, INC., Mundelein, Illinois 60060 (US)
(72) Inventor: WINDSOR, George David, Bletchingley, Surrey RH1 4LL (GB)
(74) Representative: Marchant, James Ian
(86) International application number: GB9200747
(87) International publication number: WO9218161

(56) References cited:
- WO-A-91/18627
- WO-A-92/03157
- CHEMICAL ABSTRACTS, vol. 93, no. 3, 21 July 1980, Columbus, OH (US); G.G. CHRISTIANSON et al., p. 152, no. 19975p

## Description

This invention relates to the production of vaccines, more particularly the production of vaccines against mycoplasmas.

As used herein the term "mycoplasma" refers to an organism within the order Mycoplasmatales and, in particular, members of the genera Mycoplasma and Acholeplasma within this order.

Mycoplasmas are widely distributed in nature and certain pathogenic strains are responsible for a number of diseases in man and animals. For example, respiratory diseases such as pneumonias in both man and animals are caused by such organisms and these diseases are characterised by a protracted course of the disease and high morbidity. In this connection, particular mention should be made of the disease enzootic pneumonia in pigs (EPP) which is of considerable economic significance, especially in the case of intensively reared animals, and for which the causative agent is Mycoplasma hyopneumoniae (formerly also known as M. suipneumoniae).

A number of attempts have been made to produce vaccines to protect animals against mycoplasmal infections, particularly EPP. Typically the method for the production of the vaccine involved the steps of growing the organism, for example M. hyopneumoniae, in culture to the desired yield, inactivating and harvesting the organism, and formulating the harvested cells into a vaccine optionally together with a suitable adjuvant. Mycoplasmas are generally difficult to grow in culture and in preparing a vaccine it is important to ensure that the antigenicity of the live organism is retained after inactivation. The most common method of inactivating bacteria during vaccine production is treatment with formalin and this has been the method generally used in the attempts to produce vaccines against mycoplasmas. It has not hitherto proved possible to produce a vaccine in this way which has provided satisfactory protection against EPP.

A number of different methods are also known for the inactivation of viruses during the production of vaccines against virus diseases including treatment of the live virus with ethyleneimine. However, the use of ethyleneimine presents considerable difficulties in practice as a result of the fact that it is highly toxic and can only be handled under the most stringent safety precautions.

The use of binary ethyleneimine to inactivate mycoplasmal contamination in animal sera used for the propagation of cell cultures has also been investigated (Christianson et al, J. Clin. Microbiol., ll(4), 377-379 (1980)), but the conclusion was drawn that binary ethyleneimine could not be recommended for this purpose.

The present invention provides a process for the production of a vaccine effective in protecting an animal against a condition associated with infection by an organism of the genera Mycoplasma and Acholeplasma within the order Mycoplasmatales, which comprises growing the organism in culture, inactivating the organism by treating with ethyleneimine or a suitable derivative thereof, harvesting the cells of the organism and processing the cells into a vaccine.

The method according to the present invention involves the use of ethyleneimine or a suitable derivative thereof. Suitable derivatives include acyl derivatives, for example acetyl ethyleneimine. The invention will be described hereinafter with particular reference to the use of ethyleneimine itself.

As noted above, ethyleneimine is a highly toxic and dangerous chemical and, although it can be used as such according to the invention for the inactivation of the mycoplasma, it is highly preferred to generate the ethyleneimine in situ in the culture of the organism. The method by which the ethyleneimine is generated in situ should be such that it is produced from a precursor which is safe to handle and the ethyleneimine should then itself be inactivated after it has fulfilled its function of inactivating the organism. The methods of generating and inactivating the ethyleneimine should themselves be such as not to have any significant effect on the antigenicity of the organism.

According to a particularly preferred embodiment of the invention, ethyleneimine is generated in situ by adding 2-bromo ethylamine hydrobromide (BEA) to the culture of the mycoplasma. BEA is a relatively inert solid but under mildly alkaline conditions it cyclises to form ethyleneimine. Accordingly, at the time of adding BEA it is necessary to adjust the pH of the culture to a mildly alkaline value in the range from above 7.0 to about 8.0, more preferably 7.3 to 7.7, most preferably about 7.5.

Unless steps are taken continuously to maintain the pH at this mildly alkaline level whilst the inactivation of the organism is taking place, the pH has a tendency to fall. Accordingly, after addition of the BEA, the pH of the culture should be adjusted continuously to maintain the desired mildly alkaline value for a sufficient period to ensure that the organism has been completely inactivated. Addition of alkali is necessary primarily over the first few hours, for example about the first three hours, but it may also be necessary to maintain continuous adjustment of the pH of the culture medium for a longer period, for example about 20 to 24 hours. The pH of the culture medium may be adjusted to the desired mildly alkaline value by the addition of any suitable alkali, for example an alkali metal hydroxide, preferably sodium hydroxide.

Following inactivation of the organism, the ethyleneimine may itself be inactivated by addition of a suitable reagent which converts the ethyleneimine into a form in which it can be safely handled. Examples of suitable inactivating agents include sodium thiosulphate which inactivates ethyleneimine by cleaving the ring structure of the compound and citric acid. The cells of the mycoplasma are subsequently harvested, for example by centrifugation, and to avoid any possible operator exposure to ethyleneimine the residual culture medium may be discarded into further aqueous sodium thiosulphate solution.

The present invention is applicable to any organism of the genera Mycoplasma and Acholeplasma within the order Mycoplasmatales. Examples of such organisms include M. hyopneumoniae, M. bovis, M. dispar, M. orale, M. hominis, M. arginini and A. laidlawii. The invention is particularly applicable to the production of a vaccine against EPP and the invention will be described further by way of example with particular reference to the organism M. hyopneumoniae.

A process for the production of a vaccine against the organism M. hyopneumoniae starts from a seed culture of the organism in question. Suitable cultures are available from culture collections, for example M. hyopneumoniae 10110 available from the National Collection of Type Cultures, Colindale, UK. The seed culture is grown up in a suitable medium and then inoculated into the final volume of culture medium. Depending on the scale of vaccine production, the final volume of culture medium may be from, for example, 40 to 45 litres up to about 200 litres or more and the seed culture may be grown up to a volume of from 1 to 10% of the final volume of the culture medium before it is inoculated into this final culture medium.

Suitable culture media include, for example, a commercial broth base enriched with serum and yeast extract. An energy source such as glucose and an antibiotic such as polymyxin will generally also be included. The pH should generally be in the range from 7.2 to 7.6. The organism is grown in culture under conditions of controlled pH, preferably pH 7.1 to 7.3, with the pH being regulated by the addition of an alkali such as sodium hydroxide. Growth takes place at normal incubator temperature, for example 36°C ± 1°C, and is continued until the metabolic activity of the culture starts to fall as reflected by the consumption of the alkali used to adjust pH.

Once metabolic activity of the culture starts to fall, the organism is inactivated by addition of BEA to the culture medium. For example, the BEA may be added as aqueous solution of known concentration to a final concentration in the culture medium of about 0.82g/litre corresponding to about 4mM. At the same time as or immediately prior to the addition of the BEA the pH of the culture medium is adjusted to a mildly alkaline level, generally about pH 7.5, by the addition of a suitable alkali such as sodium hydroxide.

The BEA cyclises under these conditions to produce ethyleneimine which inactivates the M. hyopneumoniae. This process of inactivation requires a period of several hours, generally around 24 hours, during which time the culture is maintained at normal incubation temperature. It is important that ethyleneimine continues to be present in the culture medium during the time required for the inactivation. In the absence of further adjustment, the pH of the culture medium tends to become more acid over time. Accordingly the pH of the culture medium should be monitored continuously during the inactivation process and maintained at the desired mildly alkaline level by the addition of further alkali as required.

Once deactivation of the organism is complete, the ethyleneimine in the culture medium is itself destroyed by the addition of an excess of a suitable reagent such as sodium thiosulphate or citric acid. For example an excess of an aqueous sodium thiosulphate solution is added to provide a concentration of about 1g/litre in the culture medium.

Following deactivation of the ethyleneimine, the cells of the organism are harvested and the residual culture medium can be added to further aqueous sodium thiosulphate solution, again preferably to a final concentration of about 1g/litre.

Following harvesting of the cells, the antigen yield can be measured by a protein estimation. The cell concentrate resulting from the harvest can be processed in a conventional manner to produce a vaccine. More particularly, the cells are diluted to the appropriate level based on antigen content, for example with physiologically buffered saline, and if desired the vaccine may be formulated using an appropriate adjuvant such as oil, saponin or DEAE dextran. The preferred adjuvant is oil.

A vaccine produced by the general method described above from the organism M. hyopneumoniae was found to retain the antigenic properties of the live organism and was used successfully to protect pigs against EPP.

Without wishing to be bound by any specific theory, it is believed that conventional methods of inactivating bacteria during the production of a vaccine (e.g. treatment with formalin) affect the surface proteins of the bacteria with the result that the antigenic properties of the bacteria may be adversely affected. In some cases the affect on the surface proteins of the bacterial cells may be such that the cells can no longer be used to produce a satisfactory vaccine and this may apply particularly in the case of bacteria such as mycoplasmas which are difficult to grow in culture.

In these circumstances, it is necessary for the production of a satisfactory vaccine to use a method of inactivating the organism which has as little adverse effect as possible on the antigenic properties of the cells. It is believed that ethyleneimine deactivates the organism by destroying the genetic material (DNA) of the organism without any significant effect on the surface proteins of the cells.

The invention is illustrated by the following Examples.

### EXAMPLE 1

BEA (0.082 grams) was added to 100ml of an actively growing culture of M. hyopneumoniae 10110 to produce a final concentration of 4mM in the culture medium. The pH was adjusted to 7.5 with aqueous sodium hydroxide (1N) and thereafter the pH was measured and adjusted in the same way as follows:

| | | |
|---|---|---|
| 1 hour | pH measured at 6.95 | adjusted to pH 7.5 |
| 2 hours | pH measured at 7.2 | adjusted to pH 7.5 |
| 3 hours | pH measured at 7.4 | adjusted to pH 7.5^{*} |
| 4 hours | pH measured at 7.45 | not adjusted |
| 5 hours | pH measured at 7.45 | not adjusted |
| 6 hours | pH measured at 7.45 | not adjusted. |

| | | |
|---|---|---|
| ^{*}pH adjustment with 0.1N sodium hydroxide. | | |

Samples were taken hourly over a period of 6 hours for dilution and plate-counting, and also after 24 hours for direct plating. No viable organisms were detected at 24 hours.

### EXAMPLE 2

M. hyopneumoniae 10110 was subcultured on a culture medium having the following composition:

| | |
|---|---|
| Broth Base | 65ml |
| Pig Serum | 15ml |
| Boiled Blood Extract | 10ml |
| Yeast Extract | 10ml |
| Cysteine HCl (1%) | 1ml |
| Glucose (20%) | 5ml |
| Phenol Red (0.4%) | 1ml |
| Ampicillin (0.5%) | 0.5ml |
| Thallous acetate (1%) | 2.5ml. |

The subculture took place in the sequence 10ml, 100ml, 400ml, 4000ml. When the 4 litre culture was showing signs of active growth (slight turbidity and movement to an acid pH), this was inoculated into 40 litres of fresh medium. The pH of this culture was maintained at pH 7.2 by addition of aqueous sodium hydroxide solution whenever a fall in pH demanded correction.

After 13 days demand for sodium hydroxide declined and 36.5g BEA in about 100ml distilled water was pumped into the culture via a sterilising filter. The pH was maintained at 7.5 by addition of aqueous sodium hydroxide solution as required. After 24 hours the culture was harvested by centrifugation and the spent culture medium was discarded into citric acid to give a 0.2% solution. An alternative procedure at this stage would have been to add sodium thiosulphate after 24 hours to a level of 1.0g/litre prior to centrifugation and to discard the spent culture medium into further aqueous sodium thiosulphate solution. This alternative procedure would have given equivalent results.

The concentrated antigen produced following centrifugation was homogenised in 300ml phosphate buffered saline (PBS), homogenised and re-centrifuged. The re-sedimented antigen was again re-suspended in 300ml PBS and stored in aliquots at -70°C.

A sufficient quantity of the antigen was emulsified with an oily adjuvant (Marcol : Arlacel) so as to give approximately 13mg/subcutaneous dose and administered to 6 pigs with 10 uninoculated controls. After 4 weeks all pigs were challenged on three successive days intranasally with 5 ml volumes of a lung homogenate containing M. hyopneumoniae organisms. Post mortem four weeks after challenge revealed extensive lesions in the control pigs with none in the vaccinated animals, i.e. complete protection from challenge was afforded by the vaccination.

## Claims

1. A process for the production of a vaccine effective in protecting an animal against a condition associated with infection by an organism of the genera Mycoplasma and Acholeplasma within the order Mycoplasmatales, which comprises growing the organism in culture, inactivating the organism by treating with ethyleneimine or a suitable derivative thereof, harvesting the cells of the organism and processing the cells into a vaccine.

2. A process as claimed in claim 1 wherein the organism is treated with ethyleneimine generated in situ in a culture of the organism.

3. A process as claimed in claim 2 wherein the ethyleneimine is generated in situ by adding 2-bromo ethylamine hydrobromide (BEA) to the culture.

4. A process as claimed in claim 3 wherein at the time of adding BEA the pH of the culture is adjusted to a mildly alkaline value in the range from above 7.0 to about 8.0.

5. A process as claimed in claim 4 wherein the pH is 7.3 to 7.7.

6. A process as claimed in claim 5 wherein the pH is about 7.5.

7. A process as claimed in any of claims 4 to 6 wherein after addition of the BEA, the pH of the culture is adjusted continuously to maintain the mildly alkaline value for a sufficient period to ensure that the organism has been completely inactivated.

8. A process as claimed in any of claims 4 to 7 wherein the pH is adjusted by addition of an alkali metal hydroxide.

9. A process as claimed in any of claims 1 to 8 wherein following inactivation of the organism, the ethyleneimine or a derivative thereof is itself inactivated by addition of a reagent which converts the ethyleneimine or derivative thereof into a form in which it can be safely handled.

10. A process as claimed in claim 9 wherein the reagent is sodium thiosulphate.

11. A process as claimed in any of claims 1 to 10 wherein the organism is M. hyopneumoniae, M. bovis, M. dispar, M. orale, M. hominis, M. arginini or A. laidlawii.

12. A process as claimed in claim 11 wherein the organism is M. hyopneumoniae.

## Patentansprüche

1. Verfahren zur Herstellung eines Impfstoffes, der wirksam zum Schutz eines Lebewesens gegen eine mit einer Infektion durch einen Organismus der Gattungen Mycoplasma und Acholeplasma aus der Ordnung Mycoplasmatales verbundenen Erkrankung ist, das umfaßt: Züchten eines Organismus in einer Kultur, Inaktivieren des Organismus durch Behandlung mit Ethylenimin oder einem geeigneten Derivat davon, Entnahme der Zellen des Organismus und Verarbeitung der Zellen zu einem Impfstoff.

2. Verfahren nach Anspruch 1, worin der Organismus mit Ethylenimin behandelt wird, das in situ in einer Kultur des Organismus erzeugt wird.

3. Verfahren nach Anspruch 2, worin das Ethylenimin in situ durch Zugabe von 2-Bromethylamin-hydrobromid (BEA) zu der Kultur erzeugt wird.

4. Verfahren nach Anspruch 3, worin zur Zeit der Zugabe des BEA der pH der Kultur auf einen schwach alkalischen Wert im Bereich von 7,0 bis ungefähr 8,0 eingestellt wird.

5. Verfahren nach Anspruch 4, worin der pH 7,3 bis 7,7 beträgt.

6. Verfahren nach Anspruch 5, worin der pH ungefähr 7,5 beträgt.

7. Verfahren nach irgendeinem der Ansprüche 4 bis 6, worin nach der Zugabe des BEA der pH der Kultur kontinuierlich eingestellt wird, um den schwach alkalischen Wert für einen ausreichenden Zeitraum aufrechtzuerhalten, um sicherzustellen, daß der Organismus vollständig inaktiviert wurde.

8. Verfahren nach irgendeinem der Ansprüche 4 bis 7, worin der pH durch Zugabe eines Alkalimetallhydroxids eingestellt wird.

9. Verfahren nach irgendeinem der Ansprüche 1 bis 8, worin nach der Inaktivierung des Organismus das Ethylenimin oder ein Derivat davon selbst durch Zugabe eines Reagens inaktiviert werden, daß das Ethylenimin oder ein Derivat davon in eine Form überführt, in der es sicher gehandhabt werden kann.

10. Verfahren nach Aspruch 9, worin das Reagens Natriumthiosulfat ist.

11. Verfahren nach irgendeinem der Ansprüche 1 bis 10, worin der Organismus M. hyopneumoniae, M. bovis, M. dispar, M. orale, M. hominis, M. arginini oder A. laidlawii ist.

12. Verfahren nach Anspruch 11, worin der Organismus M. hyopneumoniae ist.

## Revendications

1. Procédé de production d'un vaccin efficace pour protéger un animal contre un état associé à une infection par un organisme des genres Mycoplasma et Acholeplasma dans l'ordre des Mycoplasmatales, caractérisé en ce que l'on élève l'organisme en culture, on inactive l'organisme en le traitant par de l'éthylèneimine, ou un dérivé de ce composé, on récolte les cellules de l'organisme et on transforme les cellules en un vaccin.

2. Procédé suivant la revendication 1, caractérisé en ce que l'on traite l'organisme par de l'éthylèneimine engendrée in situ dans une culture de l'organisme.

3. Procédé suivant la revendication 2, caractérisé en ce que l'éthylèneimine est engendrée in situ par l'addition de bromhydrate de 2-brométhylamine (BEA) à la culture

4. Procédé suivant la revendication 3, caractérisé en ce qu'au moment de l'addition du BEA, on ajuste le pH de la culture à une valeur modérément alcaline qui varie dans la plage d'environ 7,0 à environ 8,0.

5. Procédé suivant la revendication 4, caractérisé en ce que le pH varie de 7,3 à 7,7.

6. Procédé suivant la revendication 5, caractérisé en ce que le pH est d'environ 7,5.

7. Procédé suivant l'une quelconque des revendications 4 à 6, caractérisé en ce qu'après l'addition du BEA, on ajuste continuellement le pH de la culture afin de maintenir la valeur modérément alcaline pendant une période qui suffit à assurer que l'organisme ait été totalement inactivé.

8. Procédé suivant l'une quelconque des revendications 4 à 7, caractérisé en ce que l'on ajuste le pH par l'addition d'un hydroxyde de métal alcalin.

9. Procédé suivant l'une quelconque des revendications 1 à 8, caractérisé en ce que, après l'inactivation de l'organisme, on inactive l'éthylèneimine, ou un dérivé de celle-ci, par l'addition d'un réactif qui convertit l'éthylèneimine ou son dérivé en une forme sous laquelle on peut la ou le manier avec sécurité.

10. Procédé suivant la revendication 9, caractérisé en ce que le réactif est le thiosulfate de sodium.

11. Procédé suivant l'une quelconque des revendications 1 à 10, caractérisé en ce que l'organisme est M. hyopneumoniae, M. bovis, M. dispar, M. orale, M. hominis, M. arginini ou A. laidlawii.

12. Procédé suivant la revendication 11, caractérisé en ce que l'organisme est M. hyopneumoniae.
